# EUROPEAN PATENT APPLICATION

(11) **EP 4 654 317 A1**
(43) Date of publication of application: **26.11.2025**
(21) Application number: 24860252.6
(22) Date of filing: 12.08.2024
(51) Int. Cl.: H01M 10/0567, H01M 10/42, H01M 10/052, H01M 4/525, H01M 4/505

(54) **ELECTROLYTE ADDITIVE, LITHIUM SECONDARY BATTERY ELECTROLYTE COMPRISING SAME, AND LITHIUM SECONDARY BATTERY**

(30) Priority: 31.08.2023 KR 20230115745; 03.07.2024 KR 20240087849
(71) Applicant: LG Energy Solution, Ltd., Seoul 07335 (KR)
(72) Inventor: PARK, Sung Guk, Daejeon 34122 (KR); LEE, Chul Haeng, Daejeon 34122 (KR); LEE, Jung Hoon, Daejeon 34122 (KR)
(74) Representative: Hoffmann Eitle
(86) International application number: PCT/KR2024/012003
(87) International publication number: WO 2025/048331

(57) **Abstract**

The present disclosure relates to an electrolyte additive, an electrolyte for a lithium secondary battery comprising the same and a lithium secondary battery. Specifically, the present disclosure may provide an electrolyte for a lithium secondary battery which may achieve excellent high-temperature storage characteristics and high-temperature cycle characteristics by including an electrolyte additive including a compound represented by Formula 1 capable of effectively suppressing the deterioration of a positive electrode, and of being not easily decomposed on a negative electrode, and a lithium secondary battery comprising the same.

## Description

### [TECHNICAL FIELD]

### Cross-reference to Related Applications

This application claims priority from Korean Patent Application No. 10-2023-0115745, filed on August 31, 2023 and Korean Patent Application No. 10-2024-0087849, filed on July 3, 2024, the entire contents of which are incorporated by reference herein.

### TECHNICAL FIELD

The present disclosure relates to an electrolyte additive, an electrolyte for a lithium secondary battery, and a lithium secondary battery including the same.

### [BACKGROUND ART]

As dependence on electric energy is increasing in modern society, development of a large-capacity power storage device that can stably supply power and increase production is in the spotlight.

Lithium-ion batteries are a power storage device with the highest energy density that has been commercialized among power storage devices, and thus has been applied to various fields such as small electronic products, electric vehicles (EV) and power storage devices.

In particular, high output characteristics are required for lithium ion batteries applied to electric vehicles, while maintaining cycle characteristics and performance in various environments.

The lithium-ion battery includes a positive electrode comprised of a transition metal oxide containing lithium, a negative electrode capable of storing lithium, a non-aqueous electrolyte solution comprising an organic solvent containing a lithium salt, and a separator.

Meanwhile, lithium hexafluorophosphate (LiPF₆), which is mainly used as the lithium salt is easily decomposed at high temperatures to produce Lewis acid by-products such as HF and PF₅, and the by-products react with moisture to generate more Lewis acid by-products (HF). Such Lewis acid by-products may erode a passivation film formed on the electrode and its surface, thereby causing the elution of transition metal ions from the positive electrode. In addition, the eluted transition metal ions promote the decomposition of the electrolyte solvent, thereby accelerating gas generation, or are re-deposited on the positive electrode, thereby increasing the resistance of the positive electrode, and also, are transferred to the negative electrode through the electrolyte and then deposited on the negative electrode, thereby causing the additional consumption of lithium ions due to self-discharging of the negative electrode, destruction and regeneration of a solid electrolyte interphase (SEI) film, etc., resistance increase, and the like.

Since a series of such reactions reduces the amount of available lithium ions in a battery, it may be the main cause of capacity degradation of the battery. In addition, in the case where the metal ions electrodeposited on the negative electrode grow into dendrites, they cause an internal short circuit of the battery, which leads to a reduction in the secondary battery safety.

Therefore, there is a demand for electrolyte capable of improving battery performance such as high-rate charging/discharging properties as well as safety by scavenging by-products (HF, PF₅, etc.) generated due to thermal decomposition of a lithium salt, and at the same time, by forming a stable film on the electrode surface to suppress the elution of transition metals, or by suppressing the deposition of the eluted transition metal ions on the negative electrode.

### [DISCLOSURE OF THE INVENTION]

### [TECHNICAL PROBLEM]

In order to solve the above problem, the present disclosure aims to provide an electrolyte additive and an electrolyte for a lithium secondary battery including the same capable of suppressing the deterioration of a positive electrode film while it is not easily decomposed on the negative electrode surface and remains in the electrolyte.

In addition, the present disclosure aims to provide a lithium secondary battery in which high-temperature storage and high-temperature cycle characteristics are improved by including the electrolyte for a lithium secondary battery.

### [TECHNICAL SOLUTION]

[1] The present disclosure provides an electrolyte additive including a compound represented by the following Formula 1: 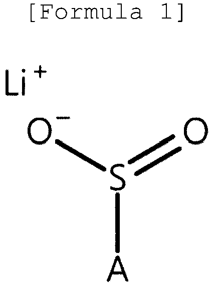 In Formula 1,
   A is a nitrogen-containing heteroaryl group having 3 to 10 carbon atoms.
[2] The present disclosure provides an electrolyte additive where in the above [1], A is a nitrogen-containing heteroaryl group having 3 to 8 carbon atoms.
[3] The present disclosure provides an electrolyte additive where in the above [1] or [2], A is a nitrogen-containing heteroaryl group having 3 to 5 carbon atoms.
[4]The present disclosure provides an electrolyte additive, wherein in at least one of the above [1] to [3], the compound represented by Formula 1 above is a compound represented by the following formula 1a or 1b: 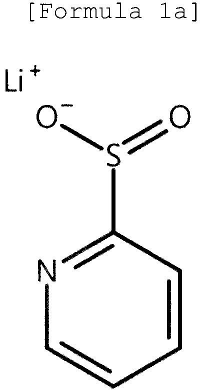 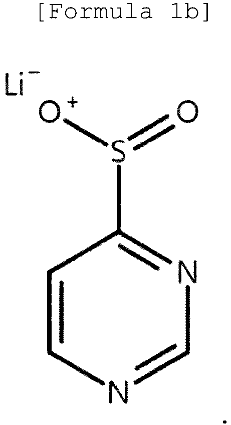
[5]The present disclosure provides an electrolyte for a lithium secondary battery including the electrolyte additive of the above [1].
[6]The present disclosure provides an electrolyte for a lithium secondary battery wherein in the above [5], the above electrolyte additive is included in an amount of 0.1 wt% to 5.0 wt% in the electrolyte for a lithium secondary battery.
[7]The present disclosure provides an electrolyte for a lithium secondary battery wherein in the above [5] or [6], the electrolyte for a lithium secondary battery further includes a lithium salt and a non-aqueous organic solvent.
[8]The present disclosure provides an electrolyte for a lithium secondary battery, wherein in at least one of the above [5] to [7], the electrolyte for a lithium secondary battery further includes at least one additional additive selected from a cyclic carbonate-based compound, a halogen-substituted carbonate-based compound, a sultone-based compound, a sulfate-based compound, a phosphate-based or phosphite-based compound, a borate-based compound, a benzene-based compound, an amine-based compound, an imidazole-based compound, a silane-based compound, or a lithium salt-based compound.
[9]The present disclosure provides a lithium secondary battery which includes a positive electrode; a negative electrode; a separator disposed between the positive electrode and the negative electrode; and the electrolyte for a lithium secondary battery according to the above [5].
[10] The present disclosure provides a lithium secondary battery which includes a lithium composite metal oxide, wherein in the above [9], the positive electrode includes a positive electrode active material, and the positive electrode active material contains lithium and at least one metal selected from nickel (Ni), cobalt (Co), manganese (Mn), or aluminum (Al).

### [ADVANTAGEOUS EFFECTS]

The electrolyte additive of the present disclosure includes a nitrogen-containing heteroaryl group and a -SO₂⁻functional group in its structure to form a durable inorganic film on the positive electrode surface, thereby suppressing the deterioration of the positive electrode and suppressing the additional oxygen release by effectively complementing oxygen vacancies that occur when the positive electrode deteriorates. Thus, if the electrolyte for a lithium secondary battery according to the present disclosure including the electrolyte additive of the present disclosure is used, a lithium secondary battery having excellent high-temperature storability and high-temperature cycle characteristics may be achieved while minimizing an increase in the resistance. The non-aqueous electrolyte of the present disclosure is particularly suitable for a high-power battery using high-capacity active materials, such as a high-nickel-based positive electrode active material or a silicon-based negative electrode material, together.

### [MODE FOR CARRYING OUT THE INVENTION]

The terms used in the present specification and the claims are used only to describe exemplary embodiments, and are not intended to limit the present disclosure.

For example, it should be appreciated that the terms such as "include," "comprise," and "have" are intended to embody specific features, numbers, steps, elements, or combinations thereof, and or allow other components to be added unless the terms are used with the terms only.

Also, in the present specification, the expression "%" denotes wt% unless explicitly stated otherwise.

Unless otherwise defined in the specification, the expression "substitution" denotes that at least one hydrogen bonded to carbon is substituted with an element other than hydrogen, wherein the element other than hydrogen may be an alkyl group having 1 to 5 carbon atoms or a fluorine element, and specifically may be an alkyl group having 1 to 5 carbon atoms.

While Lewis acid by-products and/or decomposition products, for example, hydrogen fluoride (HF), etc., formed through the hydro/thermal decomposition of conventional lithium salts, deteriorate a film formed on the electrode surface, transition metal ions may be easily eluted from the positive electrode, and the eluted transition metal ions are re-deposited on the positive electrode to be a cause of increasing the resistance of the positive electrode. In addition, after the eluted transition metal ions move to the negative electrode through the electrolyte, they are electrodeposited on the negative electrode to cause the self-discharge of the negative electrode and destruct a solid electrolyte interphase (SEI) that gives passivation ability to the negative electrode, thereby increasing the interfacial resistance of the negative electrode by promoting an additional electrolyte decomposition reaction.

Since such a series of side reactions reduce the amount of available lithium ions in the battery, this not only deteriorates the capacity of the battery, but also carries out decomposition reactions which cause electrolyte loss, thereby leading to increased resistance and failure behaviors.

Accordingly, the present disclosure aims to provide a lithium secondary battery in which the high-temperature storage and high-temperature cycle characteristics are improved by providing an electrolyte additive capable of effectively scavenging the by-products and decomposition products of electrolyte salts, which cause battery deterioration and failure behaviors and concurrently capable of continuously reducing the deterioration of a positive electrode film by not being easily decomposed and remaining in the electrolyte, and an electrolyte for a lithium secondary battery including the same.

Hereinafter, the present disclosure will be described in more detail.

The electrolyte additive according to the present disclosure, an electrolyte for a lithium secondary battery including the same, and/or a lithium secondary battery may include at least one of the configurations disclosed below, and may include any combination between technically possible configurations among the following configurations.

### Electrolyte Additive

Specifically, the present disclosure provides an electrolyte additive including a compound represented by the following Formula 1.

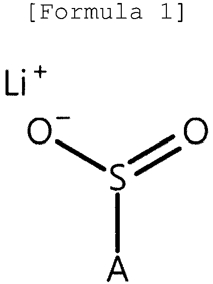

In Formula 1,
A is a nitrogen-containing heteroaryl group having 3 to 10 carbon atoms.

As most electrolyte additives are first reduced and decomposed on the negative electrode surface to form a film, there is a disadvantage of not securing the effect of forming a stable film on the positive electrode surface during operation. As a result, as high-temperature storage or high-temperature cycling progresses, the degradation of a secondary battery may occur due to the continuous degradation of the positive electrode surface. To solve this problem, the present disclosure uses an anionic compound as an additive to suppress the reduction reaction at the negative electrode, and improves the reactivity between the additive and the positive electrode by controlling the additive so that it remains in the electrolyte for an extended time.

Specifically, the compound represented by Formula 1, which is the electrolyte additive of the present disclosure, exists as an anionic compound in which a nitrogen-containing heteroaryl group in the structure is substituted with an anionic functional group,-SO₂- functional group as the Li⁺ ions contained in the structure are dissociated by the solvent. As the reduction reaction on the negative electrode surface with a relatively negative(-) potential is suppressed during operation, these anionic compounds remain in the electrolyte, and are decomposed on the positive electrode surface as the oxidation and decomposition reactivity on the positive electrode surface with a relatively positive(+) potential improves, thereby forming a durable inorganic film, such as Li₃N, NLiSO₃ or Li₂SO₄. Such inorganic films have high acid resistance, which can suppress the deterioration of the positive electrode and can also effectively complement oxygen vacancies that occur when the positive electrode deteriorates, thereby suppressing additional oxygen release.

In particular, as the compound represented by Formula 1 of the present disclosure includes a -SO₂- functional group, which is an anionic functional group in the structure, it has higher reactivity with the positive electrode compared to a compound represented by the following Formula 3 that does not contain such a functional group, thereby forming a more robust film on the positive electrode surface. Thus, the effect of suppressing a continuous degradation of the positive electrode can be achieved.

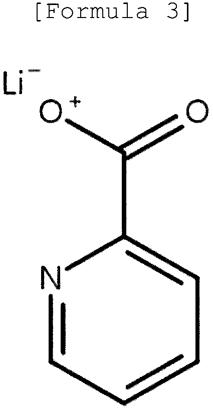

In addition, as the compound represented by Formula 1 of the present disclosure includes a nitrogen-containing heteroaryl group in the structure, the present disclosure forms a more durable Li₃N-containing film on the positive electrode surface compared to a compound represented by the following formula 4 in which the benzyl group is substituted, which can reduce the degradation of the positive electrode so that a lithium secondary battery with more improved high-temperature durability can be prepared.

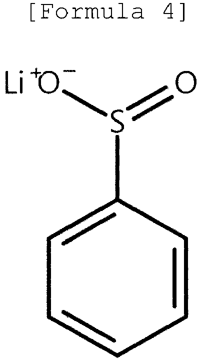

Thus, if an electrolyte for a lithium secondary battery including such an electrolyte additive is used, the effect of enhancing the high-temperature durability of a lithium secondary battery such as cycle characteristics and storage characteristics may be achieved.

Meanwhile, in the compound represented by Formula 1, the above A may be a nitrogen-containing heteroaryl group having 3 to 8 carbon atoms, and specifically may be a nitrogen-containing heteroaryl group having 3 to 5 carbon atoms.

Specifically, the compound represented by Formula 1 above may include a compound represented by Formula 1a or 1b below.

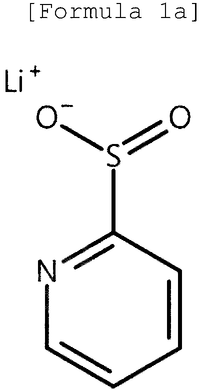

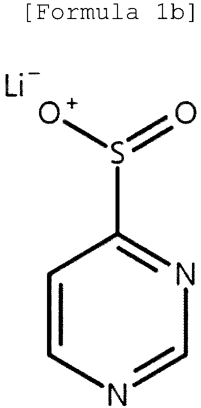

### Electrolyte for a lithium secondary battery

Further, in an embodiment, the present disclosure provides an electrolyte for a lithium secondary battery including the electrolyte additive of the present disclosure.

The electrolyte for a lithium secondary battery may further comprise a lithium salt, an organic solvent and optionally an additional additive.

### (1) Electrolyte Additive

The electrolyte for a lithium secondary battery according to the present disclosure may include an electrolyte additive including the compound represented by the above Formula 1. Since descriptions of the above compound overlap with those described above, the descriptions thereof will be omitted.

Meanwhile, the electrolyte additive may be included in an amount of 0.1 wt % to 5.0 wt % based on the total weight of the non-aqueous electrolyte, considering the effect of forming a stable film on the electrode surface and effect of scavenging the thermal decomposition products of lithium salts.

If the above electrolyte additive is included within the above content range, it may effectively suppress the elution of the transition metals of the positive electrode active materials at high temperatures by forming a robust film on the positive electrode surface and effectively scavenge the thermal decomposition products of lithium salts, while suppressing the disadvantages such as side reactions, capacity deterioration and increased resistance caused by additives as much as possible, thereby achieving excellent high-temperature durability.

If the content of the electrolyte additive is 0.1 wt% or more, the effect of scavenging the thermal decomposition products of lithium salts can be maintained even if the operation time is increased, and the effect of suppressing the elution of the transition metals can be further improved by forming a stable film on the electrode surfaces. In addition, if the content of the electrolyte additive is 5.0 wt% or less, side reactions caused by additives included in a slightly large amount can be prevented.

Specifically, the electrolyte additive may be included in an amount of 0.1 wt% to 5.0 wt%, specifically, 0.2 wt% to 4.0 wt%, more specifically 0.2 wt% to 3.0 wt%, and even more specifically 0.3 wt% to 3.0 wt% based on the total weight of the electrolyte for a lithium secondary battery.

### (2) Lithium Salt

Any lithium salt typically used in an electrolyte for a lithium secondary battery may be used without limitation as the lithium salt, and for example, the lithium salt may include Li⁺ as a cation, and may include at least one selected from F⁻, Cl⁻, Br⁻, I⁻, NO₃⁻, N(CN)₂⁻, BF₄⁻, ClO₄⁻, B₁₀Cl₁₀⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, (CF₃CF₂SO₂)₂N⁻, (CF₃SO₂)₂N⁻, (FSO₂)₂N⁻, BF₂C₂O₄⁻, BC₄O₈⁻, PF₄C₂O₄⁻, PF₂C₄O₈⁻, (CF₃)₂PF₄⁻, (CF₃)₃PF₃⁻, (CF₃)₄PF₂⁻, (CF₃)₅PF⁻, (CF₃)₆P⁻, C₄F₉SO₃⁻, CF₃CF₂SO₃⁻, CF₃CF₂(CF₃)₂CO⁻, (CF₃SO₂)₂CH⁻, CF₃(CF₂)₇SO₃⁻ or SCN⁻ as an anion.

Specifically, the lithium salt may include a single material selected from LiCl, LiBr, LiI, LiBF₄, LiClO₄, LiB₁₀Cl₁₀, LiAlCl₄, LiAlO₄, LiPF₆, LiCF₃SO₃, LiCH₃CO₂, LiCF₃CO₂, LiAsF₆, LiSbF₆, LiCH₃SO₃, LiN(SO₂F)₂ (Lithium bis(fluorosulfonyl) imide, LiFSI), LiN(SO₂CF₂CF₃)₂ (lithium bis(pentafluoroethanesulfonyl)imide, LiBETI) and LiN(SO₂CF₃)₂ (lithium bis(trifluoromethane sulfonyl)imide, LiTFSI), or a mixture of two or more thereof. In addition to these, lithium salts typically used in an electrolyte for a lithium secondary battery may be used without limitation.

The above lithium salt may be appropriately changed within a generally usable range, but may be included in the electrolyte at a concentration of 0.8M to 4.0M, and specifically, 1.0M to 3.0M, to obtain an optimal effect of forming a film for preventing the electrode surface from being corroded.

When the concentration of the lithium salt is within the above range, the viscosity of the non-aqueous electrolyte may be controlled to achieve optimal impregnation, and the mobility of lithium ions may be improved, thereby obtaining the effect of improving the capacity characteristics and cycle characteristics of a lithium secondary battery.

### (3) Non-aqueous organic solvent

In addition, a non-aqueous organic solvent will be explained as follows.

Various organic solvents typically used in a non-aqueous electrolyte may be used as the non-aqueous organic solvent without limitation, and it is not limited as long as it may minimize the decomposition due to an oxidation reaction or the like during the charging and discharging of the secondary battery and may exhibit desired characteristics with the additive.

Specifically, the non-aqueous organic solvent may include (i) a cyclic carbonate-based organic solvent, (ii) a linear carbonate-based organic solvent, or (iii) a mixture thereof.

The (i) cyclic carbonate-based organic solvent is an organic solvent having high viscosity and is an organic solvent capable of dissociating a lithium salt well in an electrolyte due to its high dielectric constant, and specific examples thereof may include at least one organic solvent selected from ethylene carbonate (EC), propylene carbonate (PC), 1,2-butylene carbonate, 2,3-butylene carbonate, 1,2-pentylene carbonate, 2,3-pentylene carbonate, or vinylene carbonate, and specifically may include at least one of ethylene carbonate and propylene carbonate.

The (ii) linear carbonate-based organic solvent is an organic solvent having low viscosity and low permittivity, wherein typical examples of the linear carbonate-based organic solvent may include at least one organic solvent selected from dimethyl carbonate (DMC), diethyl carbonate (DEC), dipropyl carbonate, ethyl methyl carbonate (EMC), methylpropyl carbonate, or ethylpropyl carbonate, and specifically it may include at least one of dimethyl carbonate and ethyl methyl carbonate.

The electrolyte for a lithium secondary battery according to the present disclosure may use (iii) a mixture of the cyclic carbonate-based organic solvent and the linear carbonate-based organic solvent to secure a high ionic conductivity, wherein the cyclic carbonate-based organic solvent and the linear carbonate-based organic solvent may be mixed in a volume ratio of 10:90 to 50:50, specifically 20:80 to 40:60.

In addition, the electrolyte for a lithium secondary battery of the present disclosure may additionally include, as the non-aqueous organic solvent, at least one organic solvent of (iv) a linear ester-based organic solvent and (v) a cyclic ester-based organic solvent having a lower melting point and higher stability at high temperatures compared to the cyclic carbonate-based organic solvent and/or the linear carbonate-based organic solvent.

Typical examples of the (iv) linear ester-based organic solvent may be at least one organic solvent selected from methyl acetate, ethyl acetate, propyl acetate, methyl propionate, ethyl propionate, propyl propionate, or butyl propionate, and specifically may include at least one of ethyl propionate and propyl propionate.

The (v) cyclic ester-based organic solvent may include at least one organic solvent selected from γ-butyrolactone, γ-valerolactone, γ-caprolactone, σ-valerolactone, or ε-caprolactone.

Meanwhile, the remainder excluding the lithium salt, electrolyte additive and additional additive as described below in the electrolyte for a lithium secondary battery may all be organic solvents unless otherwise stated.

### (4) Additional Additive

In addition, in order to prevent an electrolyte from being decomposed to cause collapse of a negative electrode in a high output environment, or further improve low-temperature high-rate discharge characteristics, high-temperature stability, overcharge protection, and a battery swelling suppression effect at high temperatures, the electrolyte for a lithium secondary battery according to the present disclosure may further include other additional additives in the above electrolyte, if necessary.

Examples of the additional additive may be at least one selected from a cyclic carbonate-based compound, a halogen-substituted carbonate-based compound, a sultone-based compound, a sulfate-based compound, a phosphate-based compound, a borate-based compound, a nitrile-based compound, a benzene-based compound, an amine-based compound, a silane-based compound, or a lithium salt-based compound.

The cyclic carbonate-based compound may include vinylene carbonate (VC) or vinyl ethylene carbonate.

The halogen-substituted carbonate-based compound may include fluoroethylene carbonate (FEC).

The sultone-based compound may include at least one compound selected from 1,3-propane sultone (PS), 1,4-butane sultone, ethane sultone, 1,3-propene sultone (PRS), 1,4-butene sultone, or 1-methyl-1,3-propene sultone.

The sulfate-based compound may include ethylene sulfate (Esa), trimethylene sulfate (TMS), or methyl trimethylene sulfate (MTMS).

The phosphate-based compound may include one or more compounds selected from lithium difluoro (bisoxalato)phosphate, lithium difluorophosphate, tris(trimethylsilyl) phosphate, tris(2,2,2-trifluoroethyl)phosphate, or tris(trifluoroethyl)phosphate.

The borate-based compound may include tetraphenylborate and lithium oxalyldifluoroborate.

The nitrile-based compound may include at least one compound selected from succinonitrile, adiponitrile, acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, or 4-fluorophenylacetonitrile.

The benzene-based compound may include fluorobenzene, and the amine-based compound may include triethanolamine or ethylenediamine, and the like.

The silane-based compound may include tetravinylsilane.

The lithium salt-based compound is a compound different from the lithium salt included in the electrolyte, and may include one or more compounds selected from LiPO₂F₂, LiODFB, LiBOB(lithium bis (oxalato) borate (LiB(C₂O₄)₂), or LiBF₄.

In a case in which vinylene carbonate, vinyl ethylene carbonate, or succinonitrile, among additional additives, is included, a more robust SEI may be formed on the surface of the negative electrode during the initial activation process of the secondary battery.

The additional additives may be used as a mixture of two or more thereof, and may be included in an amount of 30 wt% or less, specifically 0.01 wt% to 10 wt%, and preferably 0.05 wt% to 5.0 wt% based on the total weight of the electrolyte. If the content of the additional additive is less than 0.01 wt%, the effects of improving low-temperature output, high-temperature storage characteristics, and high-temperature life characteristics of the battery are insignificant, and if the content of the additional additive is greater than 30 wt%, there is a possibility that side reactions may occur excessively in the electrolyte during the charge and discharge of the battery. Particularly, if an excessive amount of the additives for forming an SEI is added, the additives may not be sufficiently decomposed at high temperatures so that they may be present in the form of unreacted materials or may precipitate in the electrolyte at room temperature. Accordingly, side reactions that degrade life or resistance characteristics of the secondary battery may occur.

### Lithium secondary battery

Further, in another embodiment of the present disclosure, the present disclosure provides a lithium secondary battery comprising the electrolyte for a lithium secondary battery of the present disclosure.

After an electrode assembly, in which the positive electrode, negative electrode, and separator between the positive electrode and the negative electrode are sequentially stacked, is formed and accommodated in a battery case, the lithium secondary battery of the present disclosure may be prepared by injecting the electrolyte of the present disclosure thereinto.

The lithium secondary battery of the present disclosure may be prepared according to a conventional method known in the art and used, and are specifically the same as those described later.

### (1) Positive Electrode

The positive electrode according to the present disclosure may include a positive electrode active material layer including a positive electrode active material, and, if necessary, the positive electrode active material layer may further include a conductive agent and/or a binder.

The positive electrode active material is a compound capable of reversibly intercalating and deintercalating lithium, wherein the positive electrode active material may specifically include a lithium composite metal oxide including lithium and at least one metal selected from nickel (Ni), cobalt (Co), manganese (Mn), or aluminum (Al). Specifically, the lithium composite metal oxide may include a compound represented by Formula 2 below.

[Formula 2] Li₁₊ₐNiₓCo_{y}M¹_{z}M²_{w}O₂

In Formula 2,
M¹ is Mn, Al or a combination thereof,
M² is at least one selected from Al, Zr, W, Ti, Mg, Ca or Sr, wherein 0≤a≤0.5, 0.55<x<1.0, 0<y≤0.4, 0<z≤0.4, 0≤w≤0.1.

1+a represents a molar ratio of lithium in a lithium transition metal oxide, and may be 0≤a≤0.5, preferably 0≤a≤0.2, and more preferably 0≤a≤0.1.

x represents a molar ratio of nickel among all transition metal elements except for lithium in the lithium transition metal oxide, and may be 0.55<x<1.0, specifically 0.6≤x≤0.98, and more specifically 0.6≤x≤0.95.

y represents a molar ratio of cobalt among all transition metal elements except for lithium in the lithium transition metal oxide, and may be 0<y≤0.4, specifically 0<y≤0.3, and more specifically 0.05≤y≤0.3.

z represents a molar ratio of the element M¹ among all transition metal elements except for lithium in the lithium transition metal oxide, and may be 0<z≤0.4, more specifically 0<z≤0.3, and even more specifically 0.01≤z≤0.3.

w represents a molar ratio of the element M² among all transition metal elements except for lithium in the lithium transition metal oxide, and may be 0<w≤0.1, more specifically 0<w≤0.05, and even more specifically 0<w≤0.02.

Specifically, the positive electrode active material may include a lithium composite transition metal oxide such as Li(Ni_{0.6}Mn_{0.2}Co_{0.2})O₂, Li(Ni_{0.7}Mn_{0.15}Co_{0.15})O₂, Li(Ni_{0.7}Mn_{0.2}Co_{0.1})O₂, Li(Ni_{0.8}Mn_{0.1}Co_{0.1})O₂, Li(Ni_{0.8}Co_{0.15}Al_{0.05})O₂, Li(Ni_{0.86}Mn_{0.07}Co_{0.05}Al_{0.02})O₂, Li(Ni_{0.90}Mn_{0.05}Co_{0.05})O₂ or Li(Ni_{0.9}Mn_{0.03}Co_{0.06}Al_{0.01})O₂ having a Ni content of 0.55 atm% or more to achieve a high capacity battery.

Further, as the positive electrode active material of the present disclosure, a lithium-manganese-based oxide (e.g., LiMnO₂, LiMn₂O₄ etc.), a lithium-cobalt-based oxide (e.g., LiCoO₂ etc.), a lithium-nickel-based oxide (e.g., LiNiO₂, etc.), a lithium-nickel-manganese-based oxide (e.g., LiNi_{1-Y}Mn_{Y}O₂(0<Y<1), LiMn_{2-z}Ni_{z}O₄(0 < Z < 2)), a lithium-nickel-cobalt-based oxide (e.g.,LiNi_{1-Y1}Co_{Y1}O₂(0<Y1<1) ), a lithium-manganese-cobalt-based oxide (e.g., LiCo_{1-Y2}Mn_{Y2}O₂(0<Y2<1), LiMn_{2-z1}Co_{z1}O₄(0 < Z1 < 2), or Li (Niₚ₁Co_{q1}Mnᵣ₂)O₄(0 <p1<2, 0 < q1 < 2, 0 < r2 < 2, p1+q1+r2=2)), etc. may be used together with a lithium composite metal oxide represented by Formula 2 above, according to the use of the secondary battery.

The positive electrode active material may be included in an amount of 80 wt% to 98 wt%, and specifically 85 wt% to 98 wt% based on the total weight of the positive electrode active material layer. When the positive electrode active material is included in an amount within the above range, excellent capacity characteristics may be exhibited.

Next, the conductive agent is used to provide conductivity to the electrode, wherein any conductive agent may be used without particular limitation as long as it has suitable electron conductivity without causing adverse chemical changes in the battery. Specific examples may include carbon black such as carbon black, acetylene black (or Denka black), Ketjen black, channel black, furnace black, lamp black, or thermal black; graphite powder such as natural graphite with a well-developed crystal structure, artificial graphite, or graphite; conductive fibers such as carbon fibers or metal fibers; conductive powder such as fluorocarbon powder, aluminum powder, or nickel powder; conductive whiskers such as zinc oxide whiskers and potassium titanate whiskers; conductive metal oxide such as titanium oxide; polyphenylene derivatives, etc. and any one thereof or a mixture of two or more thereof may be used.

The conductive agent may be included in an amount of 0.1 wt% to 10.0 wt%, preferably, 0.1 wt% to 5.0 wt% based on the total weight of the positive electrode active material layer.

Next, the binder improves the adhesion between the positive electrode active material particles and the adhesion between the positive electrode active material and a current collector.

As an example of the binder, any one of a fluorine resin-based binder including polyvinylidene fluoride (PVDF) or polytetrafluoroethylene (PTFE); a rubber-based binder including a styrene butadiene rubber (SBR), an acrylonitrile-butadiene rubber, or a styrene-isoprene rubber; a cellulose-based binder including carboxy methyl cellulose (CMC), starch, hydroxy propyl cellulose, or regenerated cellulose; a polyalcohol-based binder including polyvinyl alcohol; a polyolefin-based binder including polyethylene or polypropylene; a polyimide-based binder; a polyester-based binder; and a silane-based binder or a mixture of two or more thereof may be used.

The binder may be included in an amount of 0.1 wt% to 15.0 wt%, preferably 0.1 wt% to 10.0 wt% based on the total weight of the positive electrode active material layer.

The positive electrode of the present disclosure may be prepared by a method of preparing a positive electrode which is known in the art. For example, the positive electrode may be prepared by a method in which a positive electrode collector is coated with a positive electrode slurry, which is prepared by dissolving or dispersing the positive electrode active material, the binder, and/or the conductive agent in a solvent, dried, and then rolled, or a method in which the positive electrode active material layer is cast on a separate support, and a film separated from the support is then laminated on the positive electrode collector.

The positive electrode collector is not particularly limited as long as it has conductivity without causing adverse chemical changes in the battery, and, for example, stainless steel, aluminum, nickel, titanium, fired carbon, or aluminum or stainless steel that is surface-treated with one of carbon, nickel, titanium, silver, or the like may be used. Also, the positive electrode collector may typically have a thickness of 3 µm to 500 µm, and microscopic irregularities may be formed on the surface of the collector to improve the adhesion of the positive electrode material. The positive electrode collector, for example, may be used in various shapes such as that of a film, a sheet, a foil, a net, a porous body, a foam body, a non-woven fabric body, and the like.

The solvent may be a solvent normally used in the art, and may include dimethyl sulfoxide (DMSO), isopropyl alcohol, N-methylpyrrolidone (NMP), acetone, or water, and any one thereof or a mixture of two or more thereof may be used. An amount of the solvent used may be sufficient if the positive electrode material mixture may be adjusted to have an appropriate viscosity in consideration of a coating thickness of the positive electrode material mixture, manufacturing yield, and workability, and is not particularly limited.

### (2) Negative Electrode

Next, a negative electrode will be described.

The negative electrode according to the present disclosure includes a negative electrode active material layer including a negative electrode active material, and the negative electrode active material layer may further include a conductive agent and/or a binder, if necessary.

As the negative electrode active material, various negative electrode active materials used in the art, for example, a carbon-based negative electrode active material, a silicon-based negative electrode active material, or a mixture thereof may be used.

According to an embodiment, the negative electrode active material may include a carbon-based negative electrode active material, and, as the carbon-based negative electrode active material, various carbon-based negative electrode active materials used in the art, for example, a graphite-based materials such as natural graphite, artificial graphite, and Kish graphite; pyrolytic carbon, mesophase pitch based carbon fiber, meso-carbon microbeads, mesophase pitches, high-temperature sintered carbon such as petroleum or coal tar pitch derived cokes, soft carbon, and hard carbon may be used. A shape of the carbon-based negative electrode active material is not particularly limited, and materials of various shapes, such as an irregular shape, planar shape, flaky shape, spherical shape, or fibrous shape, may be used.

Preferably, as the negative electrode active material, at least one carbon-based negative electrode active material of natural graphite and artificial graphite may be used. The natural graphite and the artificial graphite are used together so that adhesion with the current collector may be increased to suppress exfoliation of the active material.

According to another embodiment, the negative electrode active material may include a silicon-based negative electrode active material with the carbon-based negative electrode active material.

The silicon-based negative electrode active material, for example, may include one or more selected from metallic silicon (Si), silicon oxide (SiOₓ, where 0<x≤2), silicon carbide (SiC), or a Si-Y alloy (where Y is an element selected from alkali metal, alkaline earth metal, a Group 13 element, a Group 14 element, transition metal, a rare earth element, or a combination thereof, and is not Si). The element Y may be selected from Mg, Ca, Sr, Ba, Ra, Sc, Y, Ti, Zr, Hf, Rf, V, Nb, Ta, Db (dubnium), Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Ti, Ge, P, As, Sb, Bi, S, Se, Te, Po, or a combination thereof.

Since the silicon-based negative electrode active material has higher capacity characteristics than the carbon-based negative electrode active material, better capacity characteristics may be obtained when the silicon-based negative electrode active material is further included. However, with respect to a negative electrode including the silicon-based negative electrode active material, it contains more oxygen-rich (O-rich) components in an SEI than a graphite negative electrode, and the SEI containing the O-rich components tends to be more easily decomposed when a Lewis acid, such as HF or PF₅, is present in the electrolyte. Thus, with respect to the negative electrode containing the silicon-based negative electrode active material, there is a need to suppress the formation of Lewis acids, such as HF and PF₅ in the electrolyte, or remove (or scavenge) the formed Lewis acids in order to stably maintain the SEI. Since the electrolyte according to the present disclosure includes an electrolyte additive that forms a stable film on surfaces of a negative electrode and a positive electrode, and simultaneously has the excellent effect of scavenging Lewis acids, it may effectively suppress the decomposition of the SEI when the negative electrode including the silicon-based active material is used.

Meanwhile, the carbon-based negative electrode active material and the silicon-based negative electrode active material may be mixed in a ratio of 1:99 to 97:3, preferably 85:15 to 95:5, and more preferably 90:10 to 97:3. In a case in which the mixing ratio of the silicon-based negative electrode active material to the carbon-based negative electrode active material satisfies the above range, since the volume expansion of the silicon-based negative electrode active material is suppressed while capacity characteristics are improved, excellent cycle performance may be secured.

The negative electrode active material may be included in an amount of 80 wt% to 99 wt% based on the total weight of the negative electrode active material layer. In a case in which the amount of the negative electrode active material satisfies the above range, excellent capacity characteristics and electrochemical properties may be obtained.

Next, the conductive agent is a component for further improving conductivity of the negative electrode active material, wherein the conductive agent may be added in an amount of 10 wt% or less, preferably 5 wt% or less based on the total weight of the negative electrode active material layer. Any conductive agent may be used without particular limitation so long as it has conductivity without causing adverse chemical changes in the battery, and, for example, carbon black such as carbon black, acetylene black (or Denka black), Ketjen black, channel black, furnace black, lamp black, or thermal black; graphite powder such as natural graphite with a well-developed crystal structure, artificial graphite, or graphite; conductive fibers such as carbon fibers or metal fibers; conductive powder such as fluorocarbon powder, aluminum powder, and nickel powder; conductive whiskers such as zinc oxide whiskers and potassium titanate whiskers; conductive metal oxide such as titanium oxide; or polyphenylene derivatives, may be used.

The binder is a component that assists in the binding between the conductive agent, the active material, and the current collector, wherein the binder is commonly added in an amount of 0.1 wt% to 10.0 wt% based on the total weight of the negative electrode active material layer. Examples of the binder may be a fluorine resin-based binder including polyvinylidene fluoride (PVDF) or polytetrafluoroethylene (PTFE); a rubber-based binder including a styrene butadiene rubber (SBR), an acrylonitrile-butadiene rubber, or a styrene-isoprene rubber; a cellulose-based binder including carboxy methyl cellulose (CMC), starch, hydroxy propyl cellulose, or regenerated cellulose; a polyalcohol-based binder including polyvinyl alcohol; a polyolefin-based binder including polyethylene or polypropylene; a polyimide-based binder; a polyester-based binder; and a silane-based binder.

The binder may be included in an amount of 0.1 wt% to 15.0 wt%, preferably 0.1 wt% to 10.0 wt% based on the total weight of the negative electrode active material layer.

The negative electrode may be prepared by a method of preparing a negative electrode which is known in the art. For example, the negative electrode may be prepared by a method in which a negative electrode collector is coated with a negative electrode slurry, which is prepared by dissolving or dispersing the negative electrode active material as well as optionally the binder and the conductive agent in a solvent, rolled and dried to form a negative electrode active material layer, or may be prepared by casting the negative electrode active material layer on a separate support and then laminating a film separated from the support on the negative electrode collector.

The negative electrode collector is not particularly limited as long as it has high conductivity without causing adverse chemical changes in the battery, and, for example, copper, stainless steel, aluminum, nickel, titanium, fired carbon, copper or stainless steel that is surface-treated with one of carbon, nickel, titanium, silver, or the like, and an aluminum-cadmium alloy may be used. The negative electrode collector may typically have a thickness of 3 µm to 500 µm, and, similar to the positive electrode collector, microscopic irregularities may be formed on the surface of the collector to improve the adhesion of the negative electrode active material. The negative electrode collector, for example, may be used in various shapes such as that of a film, a sheet, a foil, a net, a porous body, a foam body, a non-woven fabric body, and the like.

The solvent may be a solvent normally used in the art, and may include dimethyl sulfoxide (DMSO), isopropyl alcohol, N-methylpyrrolidone (NMP), acetone, or water, and any one thereof or a mixture of two or more thereof may be used. An amount of the solvent used may be sufficient if the negative electrode slurry may be adjusted to have an appropriate viscosity in consideration of a coating thickness of the negative electrode material mixture, manufacturing yield, and workability, and is not particularly limited.

### (3) Separator

The lithium secondary battery according to the present disclosure includes a separator between the positive electrode and the negative electrode.

The separator separates the negative electrode and the positive electrode and provides a movement path of lithium ions, wherein any separator may be used as the separator without particular limitation as long as it is typically used in a lithium secondary battery, and particularly, a separator having high moisture-retention ability for an electrolyte as well as low resistance to the transfer of lithium salt ions is preferable.

Specifically, a porous polymer film, for example, a porous polymer film prepared from a polyolefin-based polymer, such as an ethylene homopolymer, a propylene homopolymer, an ethylene/butene copolymer, an ethylene/hexene copolymer, and an ethylene/methacrylate copolymer, or a laminated structure having two or more layers thereof may be used as the separator. Also, a typical porous nonwoven fabric, for example, a nonwoven fabric formed of high melting point glass fibers or polyethylene terephthalate fibers may be used. Furthermore, a coated separator including a ceramic component or a polymer material may be used to secure heat resistance or mechanical strength, and the separator having a single layer or multilayer structure may be optionally used.

The lithium secondary battery according to the present disclosure as described above may be suitably used in portable devices, such as mobile phones, notebook computers, and digital cameras, and electric cars such as hybrid electric vehicles (HEVs).

A shape of the lithium secondary battery of the present disclosure is not particularly limited, but a cylindrical type using a can, a prismatic type, a pouch type, or a coin type may be used.

The lithium secondary battery according to the present disclosure may not only be used in a battery cell that is used as a power source of a small device, but may also be used as a unit cell in a medium and large sized battery module including a plurality of battery cells.

### Experimental Examples

### Example 1

### (Preparation of non-aqueous electrolyte for a lithium secondary battery)

LiPF₆ was dissolved in an organic solvent, in which ethylene carbonate (EC) and ethylmethyl carbonate (EMC) were mixed in a volume ratio of 30:70 such that a concentration of the LiPF₆ was 1.0M, and a non-aqueous electrolyte for a lithium secondary battery was then prepared by adding 0.2 wt% of a compound represented by Formula 1a and 0.5 wt% of vinylene carbonate (VC) (see Table 1 below).

### (Preparation of Secondary Battery)

A positive electrode active material (Li(Ni_{0.9}Mn_{0.03}Co_{0.06}Al_{0.01})O₂), a conductive agent (carbon black), and a binder (polyvinylidene fluoride) were added to N-methyl-2-pyrrolidone (NMP) as a solvent, at a weight ratio of 97.6:0.8:1.6 to prepare a positive electrode slurry (solid content: 60.0 wt%). A 13.5*µ*m thick positive electrode collector (Al thin film) was coated with the positive electrode slurry, dried, and roll-pressed to prepare a positive electrode.

A negative electrode active material (graphite: SiO=94:6 weight ratio), a binder (SBR-CMC), and a conductive agent (carbon black) were added to water as a solvent at a weight ratio of 97.6:0.8:1.6 to prepare a negative electrode slurry (solid content: 60 wt%). A 6*µ*m thick negative electrode collector (Cu thin film) was coated with the negative electrode slurry, dried, and roll-pressed to prepare a negative electrode.

After an electrode assembly was prepared by disposing the porous polypropylene separator between the positive electrode and the negative electrode, the electrode assembly was accommodated in a battery case, and the above prepared electrolyte for a lithium secondary battery was injected thereinto to prepare a lithium secondary battery.

### Example 2

A lithium secondary battery was prepared in the same manner as in Example 1 except that LiPF₆ was dissolved in an organic solvent such that a concentration of the LiPF₆ was 1.0M, and a non-aqueous electrolyte for a lithium secondary battery was prepared by adding 0.3 wt% of the compound represented by Formula 1a and 0.5 wt% of vinylene carbonate (VC) (see Table 1 below).

### Example 3

A lithium secondary battery was prepared in the same manner as in Example 1 except that LiPF₆ was dissolved in an organic solvent such that a concentration of the LiPF₆ was 1.0M, and a non-aqueous electrolyte for a lithium secondary battery was prepared by adding 0.3 wt% of the compound represented by Formula 1b and 0.5 wt% of vinylene carbonate (VC) (see Table 1 below).

### Comparative Example 1

A lithium secondary battery was prepared in the same manner as in Example 1 except that LiPF₆ was dissolved in a non-aqueous organic solvent such that a concentration of the LiPF₆ was 1.0M, and a non-aqueous electrolyte for a lithium secondary battery was prepared by adding 0.5 wt% of vinylene carbonate (VC) as an additive (as see Table 1 below).

### Comparative Example 2

A lithium secondary battery was prepared in the same manner as in Example 1 except that LiPF₆ was dissolved in a non-aqueous organic solvent such that a concentration of the LiPF₆ was 1.0M, and a non-aqueous electrolyte for a lithium secondary battery was prepared by adding 0.3 wt% of the compound represented by Formula 4 instead of the compound represented by Formula 1a and 0.5 wt% of vinylene carbonate (VC) (see Table 1 below).

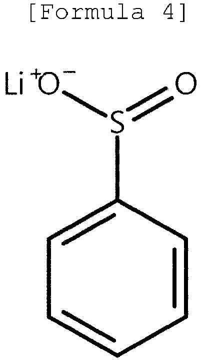

### Comparative Example 3

A lithium secondary battery was prepared in the same manner as in Example 1 except that LiPF₆ was dissolved in a non-aqueous organic solvent such that a concentration of the LiPF₆ was 1.0M, and a non-aqueous electrolyte for a lithium secondary battery was prepared by adding 0.3 wt% of the compound represented by Formula 3 instead of the compound represented by Formula 1a and 0.5 wt% of vinylene carbonate (VC) as additives (see Table 1 below).

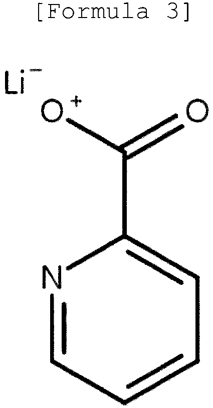

**[Table 1]**

| | Non-aqueous organic solvent | Lithi um salt | Additive | | Additio nal additiv e (wt%) |
|---|---|---|---|---|---|
| | | | Types | Content (wt%) | |
| Example 1. | EC:EMC= 30:70 volume ratio | 1.0M LiPF₆ | Formula 1a | 0.2 | VC (0.5) |
| Example 2. | | | Formula 1a | 0.3 | |
| Example 3 | | | Formula 1b | 0.3 | |
| Comparative Example 1 | | | - | - | |
| Comparative Example 2 | | | Formula 4 | 0.3 | |
| Comparative Example 3 | | | Formula 3 | 0.3 | |

Meanwhile, the abbreviation of each compound has the following meaning in Table 1.
EC: Ethylene carbonate
EMC: Ethyl methyl carbonate
VC: Vinylene carbonate

### Experimental Examples

### Experimental Example 1 Evaluation of high-temperature storage characteristics

After an activation (formation) process was performed at a 0.1 C rate for 3 hours on the lithium secondary batteries prepared in the Examples and Comparative Examples, charging (0.05 C cut-off) was performed at a 0.33 C rate to 4.2 V under a CC/CV condition at 25°C to be fully charged to SOC 100%, and they were stored at high temperatures (60°C) for 16 weeks. Subsequently, the resistance was measured by transferring them to a charger/discharger at room temperature (25°C), and the resistance increase rate was calculated using Equation 1 below, and the results are then presented in Table 2 below. Resistance increase rate(%) = { (resistance after high- temperature storage - initial resistance)/initial resistance} × 100

**[Table 2]**

| | Resistance increase rate (%) |
|---|---|
| Example 1 | 34.26 |
| Example 2 | 22.86 |

| Example 3 | 20.71 |
|---|---|
| Comparative Example 1 | 52.44 |
| Comparative Example 2 | 50.23 |
| Comparative Example 3 | 46.51 |

Referring to Table 2 above, it can be confirmed that the secondary batteries of Examples 1 to 3 of the present disclosure exhibited improved resistance increase rates (%) after high-temperature storage, compared to the lithium secondary batteries of Comparative Examples 1 to 3.

### Experimental Example 2 Evaluation of high-temperature cycle characteristics

After an activation (formation) process was performed at a 0.1 C rate for 3 hours on the lithium secondary batteries prepared in the Examples and Comparative Examples, charging (0.05 C cut-off) was performed at a 0.33 C rate to 4.2 V under a CC/CV condition at 25°C to be fully charged to SOC 100%. Charging the fully charged batteries at a 0.33 C rate to 4.2 V at 45°C under a CC/CV condition and discharging them at a 0.33C rate to 2.8 V under a CC condition was set as one cycle. After 300 cycles were performed, the capacity retention rate after 300 cycles was then measured using Equation 2 below, and the results are presented in Table 3 below. Capacity retention rate(%) = (capacity after 300 cycles/capacity after 1 cycle) × 100

**[Table 3]**

| | Capacity retention rate (%) |
|---|---|
| Example 1 | 86.35 |
| Example 2 | 89.61 |
| Example 3 | 90.72 |
| Comparative Example 1 | 81.89 |
| Comparative Example 2 | 82.76 |
| Comparative Example 3 | 85.95 |

Referring to Table 3 above, it can be confirmed that the secondary batteries of Examples 1 to 3 of the present disclosure exhibited improved capacity retention rates (%) after high-temperature cycling compared to the lithium secondary battery of Comparative Example 1 using the electrolyte, wherein the additive of the present disclosure, was not included, or the lithium secondary batteries of Comparative Examples 2 and 3, using the electrolyte wherein the compound of Formula 3 or 4 in was included the same amount instead of the additive of the present disclosure.

## Claims

1. An electrolyte additive comprising a compound represented by Formula 1: 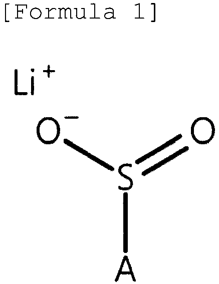 in Formula 1,
A is a nitrogen-containing heteroaryl group having 3 to 10 carbon atoms.

2. The electrolyte additive of claim 1,
wherein A is a nitrogen-containing heteroaryl group having 3 to 8 carbon atoms.

3. The electrolyte additive of claim 1,
wherein A is a nitrogen-containing heteroaryl group having 3 to 5 carbon atoms.

4. The electrolyte additive of claim 1,
wherein the compound represented by Formula 1 is a compound represented by Formula 1a or 1b:

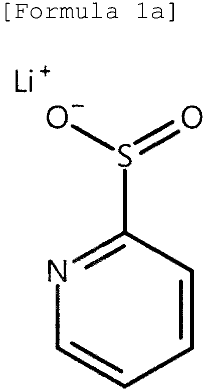

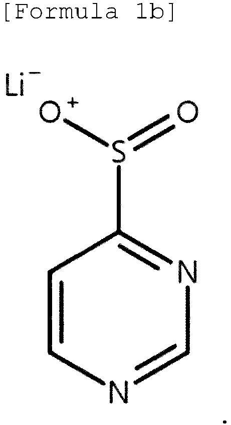

5. An electrolyte for a lithium secondary battery including the electrolyte additive of claim 1.

6. The electrolyte for a lithium secondary battery of claim 5,
wherein the electrolyte additive is included in an amount of 0.1 wt% to 5.0 wt% in the electrolyte for a lithium secondary battery.

7. The electrolyte for a lithium secondary battery of claim 5,
wherein the electrolyte for a lithium secondary battery further includes a lithium salt and an non-aqueous organic solvent.

8. The electrolyte for a lithium secondary battery of claim 5,
wherein the electrolyte for a lithium secondary battery further includes at least one additional additive selected from a cyclic carbonate-based compound, a halogen-substituted carbonate-based compound, a sultone-based compound, a sulfate-based compound, a phosphate-based or phosphite-based compound, a borate-based compound, a benzene-based compound, an amine-based compound, an imidazole-based compound, a silane-based compound, or a lithium salt-based compound.

9. A lithium secondary battery comprising a positive electrode; a negative electrode, a separator disposed between the positive electrode and the negative electrode, and the electrolyte for a lithium secondary battery of claim 5.

10. The lithium secondary battery of claim 9,
wherein the positive electrode includes a positive electrode active material, and
the positive electrode active material includes a lithium composite metal oxide including lithium and at least one metal selected from nickel (Ni), cobalt (Co), manganese (Mn), or aluminum (Al).
